(19) Europäisches Patentamt

European Patent Office

Office européen des brevets

(11) **EP 2 382 160 B1**

(12) **FASCICULE DE BREVET EUROPEEN**

(45) Date de publication et mention
de la délivrance du brevet:
**21.05.2014 Bulletin 2014/21**

(21) Numéro de dépôt: **09799186.3**

(22) Date de dépôt: **23.12.2009**

(51) Int Cl.:
*H01M 4/24* (2006.01)          *H01M 4/48* (2010.01)
*H01M 10/30* (2006.01)        *C07C 53/10* (2006.01)
*C07F 15/04* (2006.01)

(86) Numéro de dépôt international:
**PCT/IB2009/055936**

(87) Numéro de publication internationale:
**WO 2010/073227 (01.07.2010 Gazette 2010/26)**

(54) **MATIERE ACTIVE D'ELECTRODE NEGATIVE DE GENERATEUR NICKEL-CADMIUM**

AKTIVES MATERIAL FÜR DIE NEGATIVE ELEKTRODE EINER NICKEL-KADMIUM-BATTERIE

NEGATIVE ELECTRODE ACTIVE MATERIAL FOR NICKEL-CADMIUM BATTERY

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **24.12.2008 FR 0807446**

(43) Date de publication de la demande:
**02.11.2011 Bulletin 2011/44**

(73) Titulaire: **Saft Groupe S.A.
93170 Bagnolet (FR)**

(72) Inventeurs:
• **CHEVALIER, Stéphanie
F-33200 Bordeaux (FR)**
• **AUDRY, Claudette
F-33520 Bruges (FR)**
• **DENDARY, Mélanie
F-33200 Eysines (FR)**

• **DESPREZ, Philippe
F-33320 Le Taillan Medoc (FR)**
• **MARLID, Björn
S-57240 Oskarshamn (SE)**
• **SJÖVALL, Rune
S-24735 Södra Sandly (SE)**
• **GOTTFRIDSSON, Jerry
S-57240 Oskarshamn (SE)**

(74) Mandataire: **Hirsch & Associés
58, avenue Marceau
75008 Paris (FR)**

(56) Documents cités:
**WO-A- 00/69005          JP-A- 3 238 755
JP-A- 58 198 856        US-A- 3 837 919
US-A- 4 983 477**

**Description**

DOMAINE TECHNIQUE

Le domaine technique auquel se rapporte l'invention est celui des matières électrochimiquement actives utilisables dans l'électrode négative d'un générateur électrochimique ou accumulateur de type nickel-cadmium.

ETAT DE LA TECHNIQUE

Un générateur électrochimique de type nickel-cadmium comprend généralement une ou plusieurs électrodes positives ou cathode(s) contenant une matière électrochimiquement active à base d'hydroxyde de nickel ainsi qu'une ou plusieurs électrodes négatives ou anodes(s) contenant une matière électrochimiquement active à base d'oxyde de cadmium. Au moins un séparateur, généralement de polyoléfine ou de polyamide, est intercalé entre une électrode positive et une électrode négative. Les électrodes positives, négatives et le séparateur sont introduits dans un bac puis imprégnés d'un électrolyte qui est une solution concentrée d'une base forte choisie parmi KOH, LiOH, NaOH ou un mélange de celles-ci.

Des compositions d'électrodes négatives à base de cadmium pour piles alcalines de type nickel-cadmium sont décrites par exemple dans les documents WO 00/69005 A, JP 03 238755 A, US 4 983 477 A, JP 58 198856 A et US 3 837 919 A. Ces compositions comprennent un composé de cadmium tel que l'hydroxyde de cadmium ou l'oxyde de cadmium comme matière active, et un composé du nickel, de préférence l'hydroxyde de nickel, comme additif.

L'électrode négative au cadmium de ce type de générateur peut être fabriquée selon plusieurs procédés.

Dans un premier procédé, un support poreux fritté de nickel est trempé alternativement dans des solutions de sel de cadmium et de soude, permettant le remplissage des pores du fritté par de l'hydroxyde de cadmium qui constitue la matière active négative de l'accumulateur. Cette technique permet une bonne répartition volumique de la matière active et une bonne collecte du courant, d'où une forte puissance. Cette électrode dite « électrode frittée cadmium » présente l'inconvénient d'être coûteuse.

Dans un second procédé, de la poudre d'oxyde de cadmium CdO est mélangée avec différents additifs (conducteurs, stabilisants). On ajoute à ce mélange une solution aqueuse contenant un liant de façon à obtenir une pâte. Le stabilisant limite la réaction d'hydratation de CdO en $Cd(OH)_2$ à 10 à 20 %. On dépose la pâte obtenue sur un support conducteur de courant afin d'obtenir une électrode. Le support collecteur de courant est généralement un métal déployé ou un feuillard d'acier nickelé perforé. L'électrode est ensuite séchée. L'électrode obtenue est dite « électrode empâtée » ou « plastifiée ». Ce procédé est le plus utilisé pour les petits accumulateurs nickel-cadmium destinés aux applications portables ou pour les

accumulateurs industriels pour des applications de type cyclage. Ce procédé est peu coûteux et se prête bien aux fabrications en grande série mais ne permet pas d'obtenir des accumulateurs de très longue durée de vie, par exemple 15 à 20 ans.

Dans un troisième procédé, de la poudre d'oxyde de cadmium CdO est mélangée avec différents additifs conducteurs et un liant permettant l'agglomération des poudres. Les poudres sont mélangées et de l'eau additionnée de sulfate de nickel est ajoutée. On obtient une pâte où l'oxyde de cadmium est majoritairement hydraté (plus de 60% de taux d'hydratation). Cette pâte est séchée et introduite dans des pochettes perforées en feuille d'acier nickelée qui sont assemblées pour constituer les électrodes. On trouve une description détaillée de ce troisième procédé « électrode pochette » par exemple au chapitre 26 de l'ouvrage de référence « Handbook of batteries » de David Linden publié chez McGraw-Hill en 1994 (ISBN 0-07-037921-1).

Une telle solution technique présente cependant l'inconvénient d'entraîner des courts-circuits internes au générateur. En effet, les sulfates résiduels dans l'électrode négative sont relargués dans l'électrolyte et migrent vers l'électrode positive, entraînant le gonflement de cette dernière. Le gonflement augmente la pression à l'intérieur de l'électrode pochette ce qui a pour conséquence la sortie d'une partie de l'additif conducteur carbone ajouté à la matière active positive. Les ions sulfates de l'électrolyte, lorsque leur concentration dépasse environ $1,7 \times 10^{-2}$ mol.l$^{-1}$ (équivalent à 3g.l$^{-1}$ de $K_2SO_4$) sont également responsables de la formation de cristaux de $K_2SO_4$. (S. Uno FALK, Alvin J. SALKIND, Alkaline storage batteries, John Wiley and Sons, 1969). Ces cristaux recouverts de carbone conducteur sont responsables de la formation de court-circuits internes. L'utilisation de sulfate de nickel comme agent d'hydratation de l'oxyde de cadmium CdO impose donc soit un lavage des électrodes négatives pour éliminer les sulfates résiduels, soit un changement d'électrolyte dans le bac de la batterie.

On recherche donc une matière électrochimiquement active pour « électrode pochette » négative d'accumulateur nickel-cadmium présentant un rendement électrochimique initial amélioré et une bonne stabilité en cyclage. On souhaite également que les risques de courts-circuits internes liés à l'utilisation de cette matière électrochimiquement active soient minimisés. De cette façon, les étapes de lavage des électrodes négatives ou de changement de l'électrolyte pourront être supprimées.

RESUME DE L'INVENTION

A cet effet, l'invention a pour objet un composé de formule $Cd_{1-x}Ni_{,}(OH)_{2-y}(CH_3CO_2)_y$ avec $0<x\leq0,05$ et $0<y\leq0,10$.

Selon un mode de réalisation, ce composé cristallise sous la forme bêta.

Selon un mode de réalisation, le composé sous la forme cristalline bêta présente un paramètre de maille « a » compris entre 3,485 Å et 3,495 Å.

Selon un mode de réalisation, $x\geq0,01$, de préférence $x\geq0,02$, avantageusement $x\geq0,035$.

L'invention a également pour objet une matière électrochimiquement active comprenant le composé décrit ci-dessus, et l'électrode comprenant ladite matière électrochimiquement active.

Dans un mode de réalisation préféré, l'électrode est de type pochette.

L'invention a également pour objet un générateur électrochimique à électrolyte alcalin dont au moins une électrode négative est une électrode telle que décrite précédemment. De préférence, le générateur électrochimique est de type nickel-cadmium.

Selon un mode de réalisation, le générateur présente une capacité électrochimique initiale supérieure à 250 mAh/g à température ambiante, pour une charge de 7h30 au régime C/5, une décharge au régime de C/5 et une tension d'arrêt de 0,8V.

Selon un mode de réalisation, la concentration en ions sulfate dans l'électrolyte est inférieure à environ 5,7x $10^{-3}$ mol.l$^{-1}$.

L'invention a également pour objet un procédé de préparation du composé décrit ci-dessus. Ce procédé comprend les étapes suivantes :

(i) réaction d'une poudre d'oxyde de cadmium avec une solution aqueuse d'acétate de nickel,
(ii) séchage du mélange ainsi obtenu, et
(iii) éventuellement hydratation complémentaire.

Selon un mode de réalisation, le procédé comprend une étape supplémentaire de compression après l'étape (ii) de séchage ou après l'étape (iii) d'hydratation complémentaire.

L'invention a également pour objet un procédé de préparation d'une électrode, comprenant les étapes suivantes :

(i') réaction d'une poudre d'oxyde de cadmium avec une solution aqueuse d'acétate de nickel,
(ii') séchage du mélange ainsi obtenu,
(iii') compression du mélange sec,
(iv') dépôt du mélange compressé sur un collecteur de courant pour obtenir une électrode et,
(v') exposition de l'électrode obtenue à une solution aqueuse.

L'invention vise encore un procédé de préparation d'une « électrode pochette » dont la matière électrochimiquement active comprend le composé selon l'invention.

BREVE DESCRIPTION DES FIGURES

La Figure 1 est une représentation schématique de la structure cristalline hexagonale compacte de β-$Cd(OH)_2$.
La Figure 2 représente le rendement électrochimique (mAh/g) de la matière électrochimiquement active selon l'invention, en fonction de la valeur du paramètre de maille a (Å), pour différents additifs utilisés au cours de l'hydratation.

Ex.1 : $NiSO_4$ avec lavage des électrodes ou changement de l'électrolyte;
Ex.2 : $Ni(CH_3CO_2)_2$ sans lavage;
Ex.3 : $Ni(OH)_2$ sans lavage;
Ex.4 : $Ni(CHO_2)_2$ sans lavage.

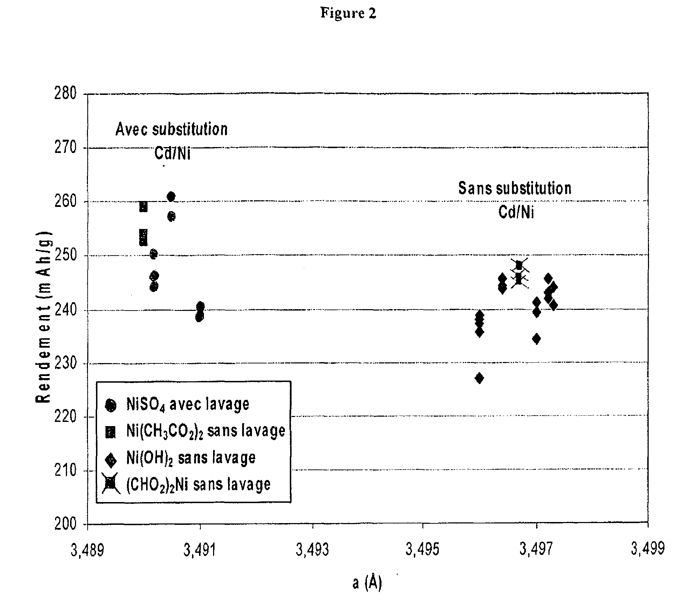

Dans les exemples de la Figure 2, le pourcentage du nombre de moles de nickel par rapport au nombre total de moles de nickel et de cadmium est de 1,9 mol%.

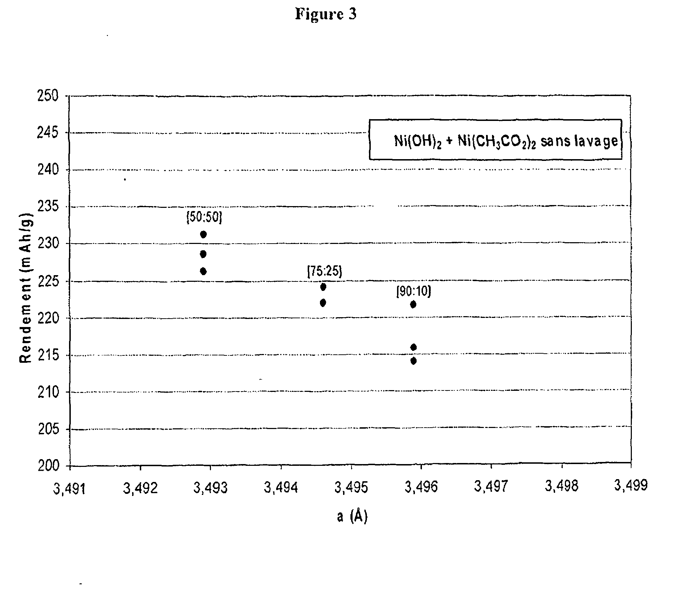

La Figure 3 représente le rendement électrochimique (mAh/g) de la matière électrochimiquement active selon l'invention, en fonction de la valeur du paramètre de maille a (Å), pour le mélange des deux additifs $Ni(OH)_2$ et $Ni(CH_3CO_2)_2$ sans lavage de l'électrode. Les indications [50:50], [75:25] et [90:10] représentent le ratio des masses de

$Ni(OH)_2$ et de $Ni(CH_3CO_2)_2$. Le pourcentage du nombre total de moles de nickel par rapport au nombre total de moles de nickel et de cadmium est de 2,8 mol%.

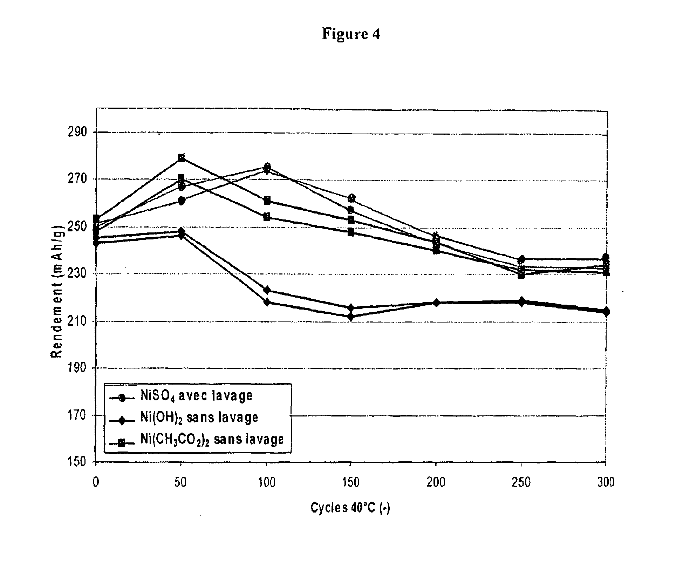

La Figure 4 représente l'évolution du rendement électrochimique (mAh/g) à 40°C de la matière électrochimiquement active selon l'invention en fonction du nombre de cycles effectués, pour différents additifs utilisés au cours de l'hydratation.

## DESCRIPTION DETAILLEE DES MODES DE REALISATION

L'invention repose sur l'utilisation d'une matière active à base d'hydroxyde de cadmium obtenue par un procédé comprenant une étape d'hydratation de l'oxyde de cadmium en présence d'un additif qui est de l'acétate de nickel $Ni(CH_3CO_2)_2$. Au cours de l'étape d'hydratation, l'oxyde de cadmium CdO se transforme en hydroxyde de cadmium $Cd(OH)_2$. L'utilisation d'acétate de nickel au cours de l'hydratation permet d'obtenir une matière active présentant un rendement électrochimique initial amélioré tout en minimisant les risques de court-circuits. On entend par rendement électrochimique initial le rendement électrochimique de la matière active négative après formation et avant cyclage. La formation électrochimique comporte un cycle de charge-décharge, la phase de charge étant réalisée à température ambiante au régime de C/5 pendant 12h30 et la phase de décharge étant réalisée à la même température au régime de C/5 jusqu'à 0,8V.

Au cours de la phase d'hydratation de l'oxyde de cadmium, une partie des ions cadmium de l'hydroxyde de cadmium est remplacée par des ions nickel. Cette substitution est possible car l'acétate de nickel est soluble en solution aqueuse et peut co-précipiter en même temps que le cadmium dans la phase d'hydratation. De même, au cours de l'hydratation, une partie des ions hydroxydes de l'hydroxyde de cadmium est remplacée par des ions acétate. Une partie des ions acétate peut également s'insérer entre les feuillets de cadmium. La réaction d'hydratation d'oxyde de cadmium en présence d'acétate de nickel permet de former le composé $Cd_{1-x}Ni_x(OH)_{2-y}(CH_2CO_2)_y$ avec $0<x\leq0,05$ et $0<y\leq0,10$.

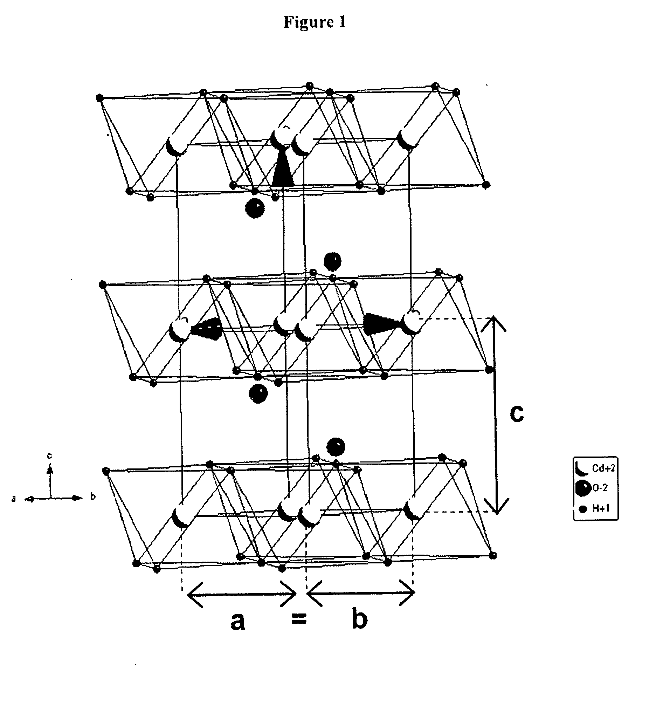

Selon un mode préféré de réalisation, le composé $Cd_{1-x}Ni_x(OH)_{2-y}(CH_3CO_2)_y$ avec $0<x\leq0,05$ et $0<y\leq0,10$ de l'invention cristallise dans la phase $\beta$. La phase cristalline $\beta$ est caractérisée par un réseau hexagonal tel que représenté à la Figure 1 dans le cas de $\beta$-$Cd(OH)_2$. La structure est de type brucite. Elle est caractérisée par trois paramètres de maille unitaire a = b et c. Le volume de la maille unitaire est donné par la relation : $V = a^2 \times c \times \sin 60°$.

La détermination du paramètre de maille peut être réalisée par diffraction des rayons X (DRX). Le rayonnement utilisé est : Cu K$\alpha$ (1,54065Å). Les paramètres de maille de l'hydroxyde $\beta$-$Cd_{1-x}Ni_x(OH)_{2-y}(CH_3CO_2)_y$ sont affinés à l'aide du logiciel d'affinement structural TOPAS R (méthode de Rietveld pour les poudres) de chez Brüker AXS. Outre $\beta$-$Cd_{1-x}Ni_x(OH)_{2-y}(CH_3CO_2)_y$, ce logiciel permet la simulation de l'ensemble des autres phases présentes dans le mélange : CdO, $\gamma$-$Cd(OH)_2$ pour les dérivés du cadmium ainsi que des phases annexes (additifs). Les corrections liées au déplacement d'échantillon dans le faisceau sont prises en compte. L'affinement est mené sur 13 raies, jusqu'à la raie (2 0 2) vers 75° en 2$\theta$. Le pas de mesure est de 0,03° et le temps de comptage est de 1,5 à 2' par pas.

L'incorporation de nickel dans le réseau cristallin de l'hydroxyde de cobalt a pour effet de réduire la valeur du paramètre «a» de maille unitaire qui passe d'une valeur comprise entre environ 3,495 à 3,499 Å pour $\beta$-$Cd(OH)_2$ à une valeur comprise entre 3,485 Å et 3,495 Å pour $\beta$-$Cd_{1-x}Ni_x(OH)_{2-y}(CH_3CO_2)_y$. Le choix de cette plage de valeur pour le paramètre de maille permet d'obtenir une matière active possédant un rendement élevé. Le paramètre de maille « a » de cette matière active dépend du taux de substitution partielle du cadmium par le nickel. Le paramètre de maille « a » suit une loi linéaire d'évolution entre la phase $\beta$-$Cd(OH)_2$ pure et la phase $\beta$-$Ni(OH)_2$ pure. Il s'agit de la loi de Végard qui est vérifiée expérimentalement. A partir de cette relation, on peut calculer le taux de substitution x de Cd par Ni dans la maille de $\beta$-$Cd(OH)_2$ en fonction de « a » :

$$x\,(\%) \approx (3,496-a)\,/\,0,0031$$

Les tests ont montré que le taux maximal de substitution x est d'environ 5%.

Selon un mode de réalisation, x est supérieur ou égal à 0,01.

Selon un mode de réalisation, x est supérieur ou égal à 0,02.

Selon un mode de réalisation, x est supérieur ou égal à 0,035.

Le procédé de fabrication du composé selon l'invention comprend les étapes suivantes :

a) on fournit une poudre d'oxyde de cadmium ;

b) on ajoute à la poudre d'oxyde de cadmium d'éventuels additifs améliorant la conductivité électronique de la matière active, tel que de la poudre de nickel métallique, du noir de carbone. On ajoute également des liants organiques ainsi qu'éventuellement des additifs de type oxydes de métaux de transition ;

c) on prépare une solution aqueuse d'acétate de nickel. De préférence, la concentration d'acétate de nickel est comprise entre 0,1 et 1 mol.l$^{-1}$ ;

d) on ajoute la solution aqueuse de l'étape c) au mélange de l'étape b). La quantité de solution ajoutée est ajustée de manière à obtenir un mélange ayant la consistance d'une pâte. L'étape de mise en contact de la solution aqueuse avec l'oxyde de cadmium constitue l'étape de préhydratation de l'oxyde de cadmium ;

e) on laisse reposer la pâte pendant une durée d'au moins environ 12 heures à température ambiante.

A cette étape, l'oxyde de cadmium est déjà presque quasiment hydraté en hydroxyde de cadmium (le taux d'hydratation de l'oxyde de cadmium est supérieur à 90%).

On sèche la pâte de l'étape e) afin d'en éliminer l'eau et obtenir une poudre. On comprime la poudre qui prend alors la forme d'une briquette. On place cette briquette entre deux plaques de collecteur de courant qui peuvent être des feuillards métalliques perforés. On parle de « montage pochette ». L'électrode ainsi obtenue est insérée dans le bac du générateur. Celui-ci est rempli d'électrolyte. Le contact entre l'oxyde de cadmium et l'électrolyte du générateur entraîne la fin de l'hydratation de l'oxyde de cadmium en hydroxyde de cadmium.

L'intérêt de l'additif d'hydratation acétate de nickel est de minimiser les risques de court-circuits internes sans lavage de l'électrode négative, tout en assurant des rendements électrochimiques équivalents à ceux obtenus avec le sulfate de nickel. Cette amélioration des rendements électrochimiques est liée à la substitution partielle de Cd par Ni dans la maille de β-Cd(OH)$_2$, obtenue pour les additifs sulfate de nickel et acétate de nickel.

L'invention concerne tous les générateurs électrochimiques à électrolyte alcalin dont au moins une électrode négative est à base de cadmium. Elle s'applique particulièrement aux générateurs électrochimiques de type nickel-cadmium à « électrodes pochettes ».

EXEMPLES

On a fabriqué différentes matières actives au cadmium en utilisant différents additifs d'hydratation. Dans tous les exemples, le ratio du nombre de moles de nickel sur le nombre total de moles de nickel et de cadmium : Ni/(Ni+Cd) est de 1,9 mol%.

Exemple 1 hors de l'invention: L'additif d'hydratation utilisé est l'hydroxyde de nickel Ni(OH)$_2$. Ni(OH)$_2$ est un composé insoluble. On n'observe donc pas de substitution du cadmium de l'hydroxyde de cadmium par l'élément nickel. Le paramètre de maille « a » vaut de 3,496 à 3,497 Å. Le rendement électrochimique initial obtenu est inférieur à 250 mAh/g. (Figure 2 et Tableau 1)

Exemple 2 selon l'invention: L'additif d'hydratation utilisé est l'acétate de nickel Ni(CH$_3$CO$_2$)$_2$. En solution aqueuse, on observe la dissolution de l'acétate de nickel. Les ions nickel provenant de l'acétate de nickel se substituent au cadmium de l'hydroxyde de cadmium. On constate par diffraction aux rayons X une réduction du paramètre de maille « a » qui se situe dans la plage allant de 3,490 à 3,491 Å. Le rendement électrochimique initial est supérieur à 250 mAh/g (Figure 2 et Tableau 1). Aucun lavage des électrodes ou changement d'électrolyte n'est nécessaire.

Exemple 3 hors de l'invention: L'additif d'hydratation utilisé est le sulfate de nickel Ni(SO$_4$). En solution aqueuse, on observe la dissolution du sulfate nickel. Les ions nickel provenant du sulfate de nickel se substituent au cadmium de l'hydroxyde de cadmium: Le paramètre de maille a vaut de 3,490 à 3,491 Å. Les ions sulfates résiduels dans la matière active sont responsables de la formation de court-circuits internes. Il est donc nécessaire de réaliser un lavage des électrodes ou un changement d'électrolyte (Figure 2 et Tableau 1)

Exemple 4 hors de l'invention: L'additif d'hydratation utilisé est le formiate de nickel Ni(CHO$_2$)$_2$. En solution aqueuse, on observe une très faible dissolution du formiate de nickel. On n'observe donc pas de substitution du cadmium de l'hydroxyde de cadmium par l'élément nickel. Le paramètre de maille « a » vaut 3,497 Å (Figure 2 et Tableau 1)

La Figure 4 montre qu'un générateur nickel-cadmium dont au moins une électrode négative contient comme matière électrochimiquement active le composé selon l'invention présente une capacité électrochimique initiale élevée, d'au moins 250 mAh/g de matière active négative. Un tel générateur présente également de bonnes performances en cyclage car le rendement après 150 cycles est d'environ 250 mAh/g. Au contraire, le générateur dont au moins une électrode négative contient comme matière électrochimiquement active un composé préparé par préhydratation avec une solution contenant Ni(OH)$_2$, présente une capacité électrochimique inférieure à 250 mAh/g.

Le tableau 1 reprend les résultats représentés sur la Figure 2 et indique en complément le taux d'hydratation initial de l'oxyde de cadmium CdO avant montage en « électrode pochette ».

Tableau 1

| Agent d'hydratation | Taux d'hydratation initial de CdO (%) | Paramètre de maille « a » de $\beta$-Cd(OH)$_2$ (Å) | Capacité électrochimique (mAh.g$^{-1}$) |
|---|---|---|---|
| NiSO$_4$ | 97 | 3,491 | 240,4 |
| | | | 238,6 |
| | | | 238,6 |
| | 87 | 3,491 | 257,2 |
| | | | 261,0 |
| | 94 | 3,490 | 250,2 |
| | | | 246,3 |
| | | | 244,0 |
| Ni(OH)$_2$ | 89 | 3,496 | 235,7 |
| | | | 238,7 |
| | | | 227,1 |
| | 82 | 3,496 | 238,0 |
| | | | 237,3 |
| | 90 | 3,497 | 239,4 |
| | | | 234,3 |
| | | | 241,3 |
| | 84 | 3,497 | 242,9 |
| | | | 241,9 |
| | | | 245,6 |
| | 74 | 3,496 | 243,9 |
| | | | 245,7 |
| | | | 244,4 |
| | 82 | 3,497 | 240,6 |
| | | | 243,9 |
| Ni(CH$_3$CO$_2$)$_2$ | 95 | 3,490 | 258,9 |
| | 96 | 3,490 | 252,7 |
| | | | 253,8 |
| Ni(CHO$_2$)$_2$ | 94 | 3,497 | 245,1 |
| | | | 246,2 |
| | | | 248,1 |

## Revendications

**1.** Composé de formule Cd$_{1-x}$Ni$_x$(OH)$_{2-y}$(CH$_3$CO$_2$)$_y$ avec $0<x\leq0,05$ et $0<y\leq0,10$.

**2.** Composé selon la revendication 1, sous la forme cristalline bêta.

**3.** Composé selon la revendication 2, présentant un paramètre de maille « a » compris entre 3,485 Å et 3,495 Å.

**4.** Composé selon l'une des revendications 1 à 3, dans lequel $x\geq0,01$, de préférence $x\geq0,02$, avantageusement $x\geq0,035$.

**5.** Electrode comprenant une matière active qui est le composé selon l'une des revendications 1 à 4.

**6.** Electrode selon la revendication 5, de type pochette.

**7.** Générateur électrochimique à électrolyte alcalin dont au moins une électrode négative est une électrode selon la revendication 5 ou 6.

**8.** Générateur électrochimique selon la revendication 7, dans lequel la concentration en ions sulfate dans l'électrolyte est inférieure à environ $5,7 \times 10^{-3}$ mol.l$^{-1}$.

**9.** Générateur électrochimique selon la revendication 7 ou 8, de type nickel-cadmium.

**10.** Procédé de préparation du composé de formule $Cd_{1-x}Ni_x(OH)_{2-y}(CH_3CO_2)_y$ avec $0<x\leq0,05$ et $0<y\leq0,10$, comprenant les étapes suivantes :

   (i) réaction d'une poudre d'oxyde de cadmium avec une solution aqueuse d'acétate de nickel,
   (ii) séchage du mélange ainsi obtenu, et
   (iii) éventuellement hydratation complémentaire.

**11.** Procédé selon la revendication 10, comprenant une étape supplémentaire de compression après l'étape (ii) de séchage ou après l'étape (iii) d'hydratation complémentaire.

**12.** Procédé de préparation selon la revendication 10 ou 11, dans lequel le composé est selon l'une des revendications 1 à 4.

**13.** Procédé de préparation d'une électrode selon la revendication 5, comprenant les étapes suivantes :

   (i') réaction d'une poudre d'oxyde de cadmium avec une solution aqueuse d'acétate de nickel,
   (ii') séchage du mélange ainsi obtenu,
   (iii') compression du mélange sec,
   (iv') dépôt du mélange compressé sur un collecteur de courant pour obtenir une électrode et,
   (v') exposition de l'électrode obtenue à une solution aqueuse.

**Patentansprüche**

**1.** Verbindung mit der Formel $Cd_{1-x}Ni_x(OH)_{2-y}(CH_3CO_2)_y$ mit $0< x\leq 0,05$ und $0 < y\leq0,10$.

**2.** Verbindung nach Anspruch 1, in ihrer beta-kristallinen Form.

**3.** Verbindung nach Anspruch 2, die einen Einheitszellenparameter "a" im Bereich von 3,485 Å bis 3,495 Å aufweist.

**4.** Verbindung nach einem der Ansprüche 1 bis 3, wobei $x \geq 0,01$, vorzugsweise $x \geq 0,02$, vorteilhafterweise $x \geq 0,035$.

**5.** Elektrode, die eine aktive Substanz umfasst, bei der es sich um die Verbindung nach einem der Ansprüche 1 bis 4 handelt.

**6.** Elektrode nach Anspruch 5, in hüllenartiger Bauform.

**7.** Elektrochemischer Stromerzeuger mit alkalischem Elektrolyten, wobei es sich bei mindestens einer seiner negativen Elektroden um eine Elektrode nach Anspruch 5 oder 6 handelt.

**8.** Elektrochemischer Stromerzeuger nach Anspruch 7, wobei die Konzentration an Sulfationen im Elektrolyten geringer als ungefähr $5,7 \times 10^{-3}$ mol.l$^{-1}$ ist.

**9.** Elektrochemischer Stromerzeuger nach Anspruch 7 oder 8, vom Typ Nickel-Cadmium.

**10.** Verfahren zur Herstellung der Verbindung mit der Formel $Cd_{1-x}Ni_x(OH)_{2y}(CH_3CO_2)_y$, mit $0 < x \leq 0,05$ und $0 < y \leq$

0,10, die folgenden Schritte umfassend:

(i) Umsetzen eines Cadmiumoxidpulvers mit einer wässrigen Lösung von Nickelacetat,
(ii) Trocknen der auf diese Weise erhaltenen Mischung, und
(iii) möglicherweise ergänzendes Hydratisieren.

11. Verfahren nach Anspruch 10, das nach dem Trocknungsschritt (ii) oder nach dem ergänzenden Hydratisierungsschritt (iii) zusätzlich einen Verdichtungsschritt umfasst.

12. Herstellungsverfahren nach Anspruch 10 oder 11, wobei die Verbindung den Ansprüchen 1 bis 4 entspricht.

13. Verfahren zur Herstellung einer Elektrode nach Anspruch 5, welches die folgenden Schritte umfasst:

(i') Umsetzen eines Cadmiumoxidpulvers mit einer wässrigen Lösung von Nickelacetat,
(ii') Trocknen der auf diese Weise erhaltenen Mischung,
(iii') Verdichten der trockenen Mischung,
(iv') Aufbringen der verdichteten Mischung auf einen Stromsammler, um eine Elektrode zu erhalten, und
(v') Einwirkenlassen einer wässrigen Lösung auf die erhaltene Elektrode.

**Claims**

1. A compound of formula $Cd_{1-x}Ni_x(OH)_{2-y}(CH_3CO_2)_y$ with $0<x\leq0.05$ and $0<y\leq0.10$.

2. The compound according to claim 1, in the crystalline beta form.

3. The compound according to claim 2, having a lattice cell parameter "a" comprised between 3.485 Å and 3.495 Å.

4. The compound according to one of claims 1 to 3, wherein $x\geq0.01$, preferably $x\geq0.02$, advantageously $x\geq0.035$.

5. An electrode comprising an active material which is the compound according to one of claims 1 to 4.

6. The electrode according to claim 5, of the envelope type.

7. An electrochemical cell with an alkaline electrolyte of which at least one negative electrode is an electrode according to claim 5 or 6.

8. The electrochemical cell according to claim 7, wherein the sulfate ion concentration in the electrolyte is less than about $5.7\times10^{-3}$ mol.l$^{-1}$.

9. The electrochemical cell according to claim 7 or 8, of the nickel cadmium type.

10. A method for preparing the compound of formula $Cd_{1-x}Ni_x(OH)_{2-y}(CH_3CO_2)_y$ with $0<x\leq0.05$ and $0<y\leq0.10$, comprising the following steps:

(i) reaction of a cadmium oxide powder with an aqueous solution of nickel acetate,
(ii) drying of the thereby obtained mixture, and
(iii) optionally additional hydration.

11. The method according to claim 10, comprising an additional compression step after the drying step (ii) or after the additional hydration step (iii).

12. The preparation method according to claim 10 or 11, wherein the compound is according to one of claims 1 to 4.

13. A method for preparing an electrode according to claim 5, comprising the following steps:

(i') reacting cadmium oxide powder with an aqueous solution of nickel acetate,
(ii') drying the thereby obtained mixture,

(iii') compressing the dry mixture,
(iv') depositing the compressed mixture on a current collector in order to obtain an electrode and,
(v') exposing the obtained electrode to an aqueous solution.

Figure 1

Figure 2

Figure 3

Figure 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0069005 A
- JP 3238755 A
- US 4983477 A
- JP 58198856 A
- US 3837919 A

**Littérature non-brevet citée dans la description**

- **DAVID LINDEN.** Handbook of batteries. Mc-Graw-Hill, 1994
- **S. UNO FALK ; ALVIN J. SALKIND.** Alkaline storage batteries. John Wiley and Sons, 1969